# EUROPÄISCHE PATENTSCHRIFT

(11) **EP 2 451 857 B1**
(45) Veröffentlichungstag und Bekanntmachung des Hinweises auf die Patenterteilung: **01.05.2013**
(21) Anmeldenummer: 10734725.4
(22) Anmeldetag: 09.07.2010
(51) Int. Cl.: C08G 18/48, C08G 65/26

(54) **VERFAHREN ZUR HERSTELLUNG VON POLYOLEN AUF BASIS NACHWACHSENDER ROHSTOFFE**
PROCESS FOR PREPARING POLYOLS FROM RENEWABLE RAW MATERIALS
PROCÉDÉ DE PRÉPARATION DE POLYOLS À PARTIR DE MATIÈRES PREMIÈRES RENOUVELABLES

(30) Priorität: 10.07.2009 EP 09165148
(43) Veröffentlichungstag der Anmeldung: 16.05.2012
(73) Patentinhaber: BASF SE, 67056 Ludwigshafen (DE)
(72) Erfinder: KUNST, Andreas, 67063 Ludwigshafen (DE); SCHELPER, Michael, 67065 Ludwigshafen (DE); TELES, Joaquim Henrique, 67165 Waldsee (DE); ELING, Berend, 49448 Lemförde (DE); REUBER, Jenny, 68167 Mannheim (DE); TEBBEN, Gerd-Dieter, Dr., 68239 Mannheim (DE)
(86) Internationale Anmeldenummer: PCT/EP2010/059883
(87) Internationale Veröffentlichungsnummer: WO 2011/004004

(56) Entgegenhaltungen:
- US-A1- 2006 229 375
- US-A1- 2007 123 725
- SELS, BERT F. ET AL.: "Solvent- and Metal-Free Ketonization of Fatty Acid Methyl Esters and Triacylglycerols with Nitrous Oxide" ADVANCED SYNTHESIS & CATALYSIS, Bd. 349, Nr. 10, 2. Juli 2007 (2007-07-02) , Seiten 1604-1608, XP002597827 Wiley DOI: DOI: 10.1002/adsc.200600645 in der Anmeldung erwähnt

## Beschreibung

Gegenstand der Erfindung ist ein Verfahren zur Herstellung von Polyolen auf Basis natürlicher Öle, insbesondere für die Herstellung von Polyurethanen.

Polyurethane werden in vielen technischen Gebieten eingesetzt. Ihre Herstellung erfolgt üblicherweise durch Umsetzung von Polyisocyanaten mit Verbindungen mit mindestens zwei mit Isocyanatgruppen reaktiven Wasserstoffatomen in Gegenwart von Treibmitteln sowie gegebenenfalls Katalysatoren und üblichen Hilfs- und/oder Zusatzstoffen.

In neuerer Zeit gewinnen Polyurethan-Ausgangskomponenten auf Basis von nachwachsenden Rohstoffen an Bedeutung. Insbesondere bei den Verbindungen mit mindestens zwei mit Isocyanatgruppen reaktiven Wasserstoffatomen können natürliche Öle und Fette zum Einsatz kommen, die üblicherweise vor dem Einsatz in Polyurethan-Anwendungen chemisch modifiziert werden, um mindestens zwei mit Isocyanatgruppen reaktive Wasserstoffatome einzuführen. Bei den chemischen Modifikationen werden zumeist natürliche Fette und/oder Öle hydroxyfunktionalisiert und gegebenenfalls in einem oder mehreren weiteren Schritten modifiziert. Als Beispiele für Anwendungen von hydroxy-funktionalisierten Fett- und/oder Ölderivaten in PU-Systemen seien beispielsweise WO 2006/116456 und WO2007/130524, genannt.

Die für den Einsatz in der Polyurethanindustrie notwendigen reaktiven Wasserstoffatome müssen wie oben beschrieben mittels chemischer Verfahren in den meisten natürlich vorkommenden Öle eingeführt werden. Hierzu existieren nach dem Stand der Technik im Wesentlichen Verfahren, die sich die in den Fettsäureestern zahlreicher Öle vorkommenden Doppelbindungen zunutze machen. Zum einen können Fette durch Umsetzung mit Percarbonsäuren in Gegenwart eines Katalysators zu den entsprechenden Fett- bzw. Fettsäureepoxiden oxidiert werden. Die anschließende säure- oder basenkatalysierte Ringöffnung der Oxiranringe in Gegenwart von Alkoholen, Wasser, Carbonsäuren, Halogenen oder Halogenwasserstoffen führt zu Bildung von hydroxyfunktionalisierten Fetten bzw. Fettderivaten (WO 2007/127379 und US 2008076901).

US 2007/0123725 A offenbart ein Verfahren zur Herstellung von Polyolen auf Basis natürlicher Öle, bei dem ungesättigte Triglyceride entweder mit Peroxycarbonsäuren oder mit Wasserstoffperoxid unter Katalyse von Carbonsäuren oxidiert werden, die gebildeten Epoxidgruppen in situ in Alkoholgruppen überführt werden und mit Alkylenoxiden unter Ringöffnung zu Polyetherpolyolen umgesetzt werden. Der Nachteil dieses Verfahrens besteht darin, dass für den ersten Reaktionsschritt (Epoxidierung) sehr korrosionsbeständige Materialen verwendet werden müssen, da dieser großindustriell mit korrosiver Perameisensäure oder mit Peressigsäure durchgeführt wird. Darüber hinaus muss die anfallende verdünnte Percarbonsäure nach der Herstellung für ein wirtschaftliches Verfahren wieder destillativ aufkonzentriert und zurückgeführt werden, was den Einsatz korrosionsbeständiger und damit energie- und kostenintensiver Destillationsapparaturen erforderlich macht.

Eine weitere Möglichkeit der Hydroxyfunktionalisierung besteht darin, das ungesättigte Fett bzw. Fettsäurederivat im ersten Reaktionsschritt in Gegenwart eines Cobalt- oder Rhodium-haltigen Katalysator mit einem Gemisch aus Kohlenmonoxid und Wasserstoff (Synthesegas) zunächst zu hydroformylieren und anschließend die mit diesem Reaktionsschritt eingeführten Aldehydfunktionen mit einem geeigneten Katalysator (z.B. Raney-Nickel) zu Hydroxygruppen zu hydrieren (vgl. WO 2006/12344 A1 oder auch J. Mol. Cat. A, 2002, 184, 65 und J. Polym. Environm. 2002, 10, 49). Bei diesem Reaktionsweg ist jedoch zu beachten, dass zumindest auch für den ersten Reaktionsschritt der Hydroformylierung die Verwendung eines Katalysators und eines Lösungsmittels notwendig ist, die für eine wirtschaftliche Herstellung ebenfalls wieder zurückgewonnen und aufgereinigt bzw. regeneriert werden müssen.
EP1170274A1 beschreibt ein Verfahren zur Herstellung von Hydroxyölen, indem ungesättigte Öle in Gegenwart von Luftsauerstoff oxidiert werden. Nachteilig ist, dass mit diesem Verfahren keine hohen Funktionalisierungsgrade erreicht werden können und dass die Umsetzungen bei hohen Temperaturen erfolgen muss, was zur teilweisen Zersetzung der Fettstruktur führt.

Eine weitere Möglichkeit, Hydroxyfunktionen in Fette einzuführen, besteht darin, dass Fett bzw. das Fettderivat in Gegenwart von Ozon zu spalten, und anschließend zum Hydroxyfettderivat zu reduzieren (vgl. Biomacromolecules 2005, 6, 713; J. Am. Oil Chem. Soc. 2005, 82, 653 und J. Am. Oil Chem. Soc. 2007, 84, 173). Auch dieser Prozess muss in einem Lösungsmittel erfolgen und wird üblicherweise bei niedrigen Temperaturen (-10 bis 0°C) durchgeführt, was ebenfalls in vergleichsweise hohe Fertigungskosten resultiert. Die sicherheitstechnischen Charakteristika dieses Prozesses erfordern darüber hinaus die kostenintensive Bereitstellung von Sicherheitsmaßnahmen, wie Mess- und Regeltechnik oder Kammerung.

In Adv. Synth. Catal. 2007, 349, 1604 wird die Ketonisierung von Fetten mittels Lachgas beschrieben. Die Ketongruppen können in Hydroxylgruppen umgewandelt werden. Es findet sich jedoch keinerlei Hinweis auf die Weiterverarbeitung dieser Produkte.

Eine Möglichkeit der Herstellung von Polyolen auf Basis nachwachsender Rohstoffe für Polyurethane besteht darin, ungesättigte natürlich vorkommende Fette wie z.B. Sojabohnenöl, Sonnenblumenöl, Rapsöl, etc. oder entsprechende Fettderivate wie Fettsäuren oder deren Monoester durch entsprechende Derivatisierung zu hydroxyfunktionalisierten Fetten bzw. Fettsäurederivaten umzusetzen. Diese Materialien können entweder direkt für die entsprechende PU-Anwendung verwendet werden oder alternativ nach zusätzlicher Anlagerung von Alkylenoxiden an die OH-Funktionen im hydroxyfunktionalisiertem Fett bzw. Fettderivat. Beispiele für die Umsetzung von Hydroxyfettderivaten mit Alkylenoxiden und der Einsatz der Reaktionsprodukte in Polyurethananwendungen sind beispielsweise in WO 2007/143135 und EP1537159 zu finden. Die Anlagerung erfolgt hierbei in den meisten Fällen mit Hilfe sogenannte Doppelmetallcyanid-Katalysatoren.

Die Aufgabe der vorliegenden Erfindung war es, Polyole auf Basis nachwachsender Rohstoffe, insbesondere auf Basis natürliche Fette und Fettsäurederivate, für Polyurethananwendungen bereitzustellen, die kostengünstig erhältlich sind und bei denen durch sehr einfache Anpassung der Reaktionsparameter unterschiedlichste Funktionalitäten abgedeckt werden können und die Produkte somit für einen breiten Anwendungsbereich zur Verfügung stehen. Insbesondere sollte die Herstellung der Öle und Fette nach einem einfachen Verfahren ohne die Verwendung kostspieliger Rohstoffe (Katalysatoren und Lösungsmittel) möglich sein.

Die Aufgabe konnte gelöst werden, indem ungesättigte natürliche Fette wie Sojabohnenöl, Sonnenblumenöl, Rapsöl, oder entsprechende Fettsäurederivate in einem ersten Schritt in Gegenwart von Distickstoffmonoxid, auch als Lachgas bezeichnet, zu ketonisierten Fetten bzw. Fettsäurederivaten oxidiert werden, und diese in einem weiteren Reaktionsschritt in Gegenwart von Hydrierungsreagenzien und optional in Gegenwart eines geeigneten Katalysators zu Hydroxyfetten reduziert werden. Die Hydroxylgruppen werden in einem weiteren Verfahrensschritt mit Alkylenoxiden umgesetzt.

Gegenstand der Erfindung ist demzufolge ein Verfahren zur Herstellung von Polyolen auf Basis nachwachsender Rohstoffe, umfassend die Schritte
a) Umsetzung von ungesättigten natürlichen Fetten, ungesättigten natürlichen Fettsäuren und/oder Fettsäureestern mit Distickstoffmonoxid,
b) Umsetzung des in Schritt a) erhaltenen Produkts mit einem Hydrierungsreagenz,
c) Umsetzung des Reaktionsprodukts aus Schritt b) mit Alkylenoxiden.

Diese Materialien können direkt als Polyolkomponente in verschiedensten Anwendungen, z.B. in der entsprechenden PU-Anwendung eingesetzt werden.

Bevorzugte sind die natürlichen, ungesättigten Fette, ausgewählt aus der Gruppe, enthaltend Rizinusöl, Traubenkernöl, Schwarzkümmelöl, Kürbiskernöl, Borretschsamenöl, Sojaöl, Weizenkeimöl, Rapsöl, Sonnenblumenöl, Erdnussöl, Aprikosenkernöl, Pistazienkernöl, Mandelöl, Olivenöl, Macadamianussöl, Avocadoöl, Sanddornöl, Sesamöl, Hanföl, Haselnussöl, Nachtkerzenöl, Wildrosenöl, Distelöl, Walnussöl, Palmöl, Fischöl, Kokosnussöl, Tallöl, Maiskeimöl, Leinsamenöl.

Bevorzugte sind die Fettsäuren und Fettsäureester ausgewählt aus der Gruppe, enthaltend Myristoleinsäure, Palmitoleinsäure, Olsäure, Vaccensäure, Petroselinsäure, Gadoleinsäure, Erucasäure, Nervonsäure, Linolsäure, α- und γ-Linolensäure, Stearidonsäure, Arachidonsäure, Timnodonsäure, Clupanodonsäure und Cervonsäure, sowie deren Estern.

Als Fettsäureester können sowohl voll- als auch teilweise veresterte ein- oder mehrwertige Alkohole verwendet werden. Als ein- oder mehrwertige Alkohole kommen Methanol, Ethanol, Propanol, iso-Propanol, Butanol, Ethylenglykol, Propylenglykol, Diethylenglykol, Dipropylenglykol, Glycerin, Trimethylolpropan, Pentaerythrit, Sorbitol, Saccharose und Mannose in Betracht.

Besonders bevorzugt sind die natürlichen, ungesättigten Fette ausgewählt aus der Gruppe, enthaltend Rizinusöl, Soja-, Palm-, Sonnenblumen- und Rapsöl. Insbesondere werden Soja-, Palm-, Sonnenblumen- und Rapsöl, eingesetzt. Diese Verbindungen werden im großtechnischen Maßstab insbesondere auch für die Biodieselproduktion verwendet.

Neben den genannten Ölen können auch solche Öle verwendet werden, die aus gentechnisch veränderten Pflanzen erhalten wurden und eine andere Fettsäurezusammensetzung aufweisen. Neben den genannten Ölen können, wie oben beschrieben, ebenfalls die entsprechenden Fettsäuren oder Fettsäureester verwendet werden.

Die Reaktionsschritte a) bis c) können unabhängig voneinander und gegebenenfalls auch zeitlich und örtlich getrennt durchgeführt werden. Es ist jedoch möglich, drei Verfahrensschritte unmittelbar nacheinander durchzuführen. Dabei ist es auch möglich, das Verfahren völlig kontinuierlich durchzuführen.

Schritt a) wird vorzugsweise unter Druck, insbesondere in einem Druckbereich von 10-300 bar und erhöhter Temperatur, insbesondere in einem Temperaturbereich von 200 bis 350 °C, durchgeführt. Dabei kann das Öl bzw. Fett in Substanz oder in Lösungen von geeigneten Lösungsmitteln, wie Cyclohexan, Aceton oder Methanol, eingesetzt werden. Die Umsetzung kann in einem Rührreaktor beliebiger Bauform oder einem Rohrreaktor stattfinden; eine Umsetzung in einem beliebigen anderen Reaktorsystemen ist prinzipiell möglich. Das verwendete Lachgas kann als Reinstoff oder als Mischung mit unter den Reaktionsbedingungen inerten Gasen, wie Stickstoff, Helium, Argon oder Kohlendioxid verwendet werden. Dabei beträgt die Menge der inerten Gase maximal 50 Vol.-%.

Nach Beendigung der Reaktion wird das Reaktionsgemisch für die weitere Verarbeitung abgekühlt, wenn notwendig das Lösungsmittel entfernt, beispielsweise mittels Destillation oder Extraktion, und dem Schritt b)mit oder ohne weitere Aufarbeitung zugeführt.

Das Reaktionsprodukt aus Schritt a) wird in Schritt b) hydriert. Auch dies erfolgt nach üblichen und bekannten Verfahren. Dazu wird die vorzugsweise gereinigte organische Phase aus Schritt a), vorzugsweise in Gegenwart eines geeigneten Lösungsmittels, mit einem Hydrierungsreagenz umgesetzt. Wird Wasserstoff als Hydrierungsreagenz verwendet, ist die Anwesenheit eines Katalysators erforderlich. Hierzu wird dann die organische Phase, bei einem Druck von 50 bis 300 bar, insbesondere bei 90 bis 150 bar und einer Temperatur von 50 bis 250 °C, insbesondere 50 bis 120 °C in Anwesenheit von Hydrierkatalysatoren umgesetzt. Als Hydrierkatalysatoren können homogene oder vorzugsweise heterogene Katalysatoren eingesetzt werden. Vorzugsweise werden Ruthenium enthaltende Katalysatoren verwendet. Die Katalysatoren können außerdem aus anderen Metallen bestehen, beispielsweise aus Metallen der Gruppe 6-11 wie z.B. Nickel, Kobalt, Kupfer, Molybdän, Palladium oder Platin. Die Katalysatoren können wasserfeucht sein. Die Hydrierung wird vorzugsweise im Festbett durchgeführt.

Neben der Verwendung von Wasserstoff als Hydrierungsreagenz in Schritt b) können beispielsweise auch komplexe Hydride wie z.B. Lithiumaluminiumhydrid, Natrium- oder Lithiumborhydrid eingesetzt werden. Dies ist beispielsweise im Organikum - Organisch-chemisches Grundpraktikum, VEB Deutscher Verlag der Wissenschaften, Berlin 1967, 6. Auflage, S. 481-484 beschrieben. In diesem Fall ist die Anwesenheit eines wasserfreien Lösungsmittels erforderlich. Als Lösungsmittel kommen alle gängigen Lösungsmittel in Frage, die nicht mit dem Hydrierungsreagenz reagieren. Beispielsweise können Alkohole wie Methanol, Ethanol, n-Propanol, iso-Propanol oder Butanol verwendet werden. Weitere Lösungsmittel sind lineare oder cyclische Ether wie Tetrahydrofuran oder Diethylether.

Nach der Hydrierung werden die organischen Lösungsmittel, falls verwendet der Katalysator und falls erforderlich Wasser abgetrennt. Das Produkt wird, sofern erforderlich, gereinigt.

Das so erhaltene Produkt wird in einem weiteren Verfahrensschritt c) mit Alkylenoxiden umgesetzt.

Die Umsetzung mit den Alkylenoxiden erfolgt üblicherweise in Anwesenheit von Katalysatoren. Hierbei können prinzipiell alle Alkoxylierungskatalysatoren zum Einsatz kommen, beispielsweise Alkalimetallhydroxide oder Lewissäuren. Bevorzugt kommen jedoch Multimetallcyanidverbindungen, so genannte DMC-Katalysatoren, zum Einsatz.

Die eingesetzten DMC-Katalysatoren sind allgemein bekannt und beispielsweise in EP 654 302, EP 862 947 und WO 00/74844 beschrieben.

Die Umsetzung mit Alkylenoxiden wird üblicherweise mit einer DMC-Konzentration von 10 - 1000 ppm, bezogen auf das Endprodukt, durchgeführt. Besonders bevorzugt wird die Reaktion mit einer DMC-Konzentration von 20 - 200 ppm durchgeführt. Ganz besonders bevorzugt wird die Reaktion mit einer DMC-Konzentration von 50 - 150 ppm durchgeführt.

Die Anlagerung der Alkylenoxide erfolgt bei den üblichen Bedingungen, bei Temperaturen im Bereich von 60 bis 180°C, bevorzugt zwischen 90 bis 140°C, insbesondere zwischen 100 bis 130°C und Drücken im Bereich von 0 bis 20 bar, bevorzugt im Bereich von 0 bis 10 bar und insbesondere im Bereich von 0 bis 5 bar. Die Mischung aus Startsubstanz und DMC-Katalysator kann vor Beginn der Alkoxylierung gemäß der Lehre von WO 98152689 durch Strippen vorbehandelt werden.

Vor der Anlagerung der Alkylenoxide werden die Produkte aus Schritt b) zumeist einer Trocknung unterworfen. Dies erfolgt zumeist durch Strippen, beispielsweise unter Verwendung von inerten Gasen, wie Stickstoff oder Wasserdampf, als Stripgase.

Als Alkylenoxide können alle bekannten Alkylenoxide verwendet werden, beispielsweise Ethylenoxid, Propylenoxid, Butylenoxid, Styroloxid. Insbesondere werden als Alkylenoxide Ethylenoxid, Propylenoxid und Mischungen aus den genannten Verbindungen eingesetzt.

In einer Ausführungsform der Erfindung werden die genannten Alkylenoxide im Gemisch mit Monomeren eingesetzt, die keine Alkylenoxide sind. Beispiele hierfür sind cyclische Anhydride, Lactone, Cyclische Ester, Kohlendioxid oder Oxetane. Bei der Verwendung von Lactonen als Comonomere sollte die Reaktionstemperatur bei der Anlagerung der Alkylenoxide > 150°C betragen.

Die oxidierten und hydrierten natürlichen Fette oder Fettderivate aus Verfahrensschritt b) können vorzugsweise allein mit den Alkylenoxiden umgesetzt werden.

Es ist jedoch auch möglich, die Umsetzung mit den Alkylenoxiden im Beisein von so genannten Co-Startern durchzuführen. Als Co-Starter können vorzugsweise Alkohole verwendet werden, wie höherfunktionelle Alkohole, insbesondere Zuckeralkohole, beispielsweise Sorbit, Hexit und Sucrose, zumeist jedoch zwei- und/oder dreifunktionelle Alkohole oder Wasser, entweder als Einzelsubstanz oder als Gemisch aus mindestens 2 der genannten Co-Starter. Beispiele für zweifunktionelle Startsubstanzen sind Ethylenglykol, Diethylenglykol, Propylenglykol, Dipropylenglykol, Butandiol-1,4 und Pentandiol-1,5. Beispiele für dreifunktionelle Startsubstanzen sind Trimethylolpropan, Pentaerythrit und insbesondere Glyzerin. Die Startsubstanzen können auch in Form von Alkoxylaten, insbesondere solche mit einem Molekulargewicht Mn im Bereich von 62 bis 15000 g/mol zum Einsatz kommen. Prinzipiell ist hierbei auch die Verwendung von Ricinusöl oder alkoxyliertem Ricinusöl möglich.

Die Anlagerung der Alkylenoxide bei der Herstellung der für das erfindungsgemäße Verfahren eingesetzten Polyetheralkohole kann nach den bekannten Verfahren erfolgen. So ist es möglich, dass zur Herstellung der Polyetheralkohole nur ein Alkylenoxid eingesetzt wird. Bei Verwendung von mehreren Alkylenoxiden ist eine so genannte blockweise Anlagerung, bei der die Alkylenoxide einzeln nacheinander angelagert werden, oder eine sogenannte statistische Anlagerung, auch als Heteric bezeichnet, bei der die Alkylenoxide gemeinsam zudosiert werden, möglich. Es ist auch möglich, bei der Herstellung der Polyetheralkohole sowohl blockweise als auch statistische Abschnitte in die Polyetherkette einzubauen. Des Weiteren sind gradientenartige oder alternierende Anlagerungen möglich, wie sie beispielsweise in DE 19960148 beschrieben wurde.

In einer Ausführungsform der Erfindung werden die Starter während der Reaktion kontinuierlich der Reaktion zugeführt. Diese Ausführungsform ist beispielsweise in WO 98/03571 beschrieben. Es ist auch möglich, die gegebenenfalls mitverwendeten Co-Starter kontinuierlich zu dosieren. Weiterhin ist es möglich, die gesamte Umsetzung mit den Alkylenoxiden kontinuierlich durchzuführen, wie ebenfalls in WO 98/03571 beschrieben.

In einer weiteren Ausführungsform der Erfindung kann die Alkoxylierung auch als sogenannter Heel-Prozess durchgeführt werden. Das bedeutet, dass das Reaktionsprodukt als Ausgangsprodukt wieder im Reaktor vorgelegt wird.

Nach Beendigung der Anlagerung der Alkylenoxide wird der Polyetheralkohol nach üblichen Verfahren aufgearbeitet, indem die nicht umgesetzten Alkylenoxide sowie leicht flüchtige Bestandteile entfernt werden, üblicherweise durch Destillation, Wasserdampf- oder Gasstrippen und oder anderen Methoden der Desodorierung. Falls erforderlich, kann auch eine Filtration erfolgen.

Die erfindungsgemäßen Polyetheralkohole aus dem Prozessschritt c) haben vorzugsweise eine mittlere Funktionalität von 2 bis 6, insbesondere von 2 bis 4 und eine Hydroxylzahl im Bereich zwischen 20 und 120 mg KOH/g. Somit eignen sie sich insbesondere für PU-Weichschaum als auch für PU-Klebstoffe, -Dichtstoffe und -Elastomere.

Die erfindungsgemäßen Polyetheralkohole aus dem Prozessschritt b) haben abhängig von der Art des verwendeten Fetts oder Fettderivates in Prozessschritt a) eine mittlere Funktionalität von 2 bis 6, insbesondere von 2 bis 4 und eine Hydroxylzahl im Bereich zwischen 50 und 300 mg KOH/g. Die Strukturen eignen sich insbesondere für die Herstellung von Polyurethanen, insbesondere für Polyurethan-Weichschaumstoffen, Polyurethan-Hartschaumstoffen und Polyurethan-Beschichtungen. Bei der Herstellung von Polyurethan-Hartschaumstoffen und Polyurethan-Beschichtungen ist es prinzipiell auch möglich, solche Polyole einzusetzen, an die keine Alkylenoxide angelagert wurden, das heißt, Polyole auf Basis von nachwachsenden Rohstoffen, bei deren Herstellung nur die Verfahrensschritte a) und b) durchgeführt wurden. Bei der Herstellung von Polyurethan-Weichschaumstoffen führen derartige Verbindungen auf Grund ihrer geringen Kettenlängen zu einer unerwünschten Vernetzung und sind daher weniger geeignet.

Die Herstellung der Polyurethane erfolgt durch Umsetzung der nach dem erfindungsgemäßen Verfahren hergestellten Polyetheralkohole mit Polyisocyanaten.

Die Herstellung der erfindungsgemäßen Polyurethane erfolgt durch Umsetzung von Polyisocyanaten mit Verbindungen mit mindestens zwei mit Isocyantgruppen reaktiven Wasserstoffatomen. Bei der Herstellung der Schaumstoffe erfolgt die Umsetzung in Anwesenheit von Treibmitteln.

Zu den eingesetzten Ausgangsverbindungen ist im Einzelnen folgendes zu sagen.

Als Polyisocyanate kommen die an sich bekannten aliphatischen, cycloaliphatischen, araliphatischen und vorzugsweise aromatischen mehrwertigen Isocyanate in Betracht.

Im einzelnen seien beispielhaft genannt: Alkylendiisocyanate mit 4 bis 12 Kohlenstoffatomen im Alkylenrest, wie z.B. Hexamethylen-diisocyanat-1,6; cycloaliphatische Diisocyanate, wie z.B. Cyclohexan-1,3- und -1,4-diisocyanat sowie beliebige Gemische dieser Isomeren, 2,4- und 2,6-Hexahydrotoluylen-diisocyanat sowie die entsprechenden Isomerengemische, 4,4'-, 2,2'- und 2,4'-Dicyclohexylmethan-diisocyanat sowie die entsprechenden Isomerengemische, araliphatische Diisocyanate, wie z.B. 1,4-Xylylen-diisocyanat und Xylylen-diisocyanat-Isomerengemische, vorzugsweise jedoch aromatische Di- und Polyisocyanate, wie z.B. 2,4- und 2,6-Toluylen-diisocyanat (TDI) und die entsprechenden Isomerengemische, 4,4'-, 2,4'- und 2,2'-Diphenylmethan-diisocyanat (MDI) und die entsprechenden Isomerengemische, Mischungen aus 4,4'- und 2,4'-Diphenylmethan-diisocyanaten, Polyphenyl-polymethylen-polyisocyanate, Mischungen aus 4,4'-, 2,4'- und 2,2'-Diphenylmethan-diisocyanaten und Polyphenyl-polymethylenpolyisocyanaten (Roh-MDI) und Mi-schungen aus Roh-MDI und Toluylendiisocyanaten. Die organischen Di- und Polyisocyanate können einzeln oder in Form von Mischungen eingesetzt werden.

Häufig werden auch sogenannte modifizierte mehrwertige Isocyanate, d.h. Produkte, die durch chemische Umsetzung organischer Di- und/oder Polyisocyanate erhalten werden, verwendet. Beispielhaft genannt seien Isocyanurat- und/oder Urethangruppen enthaltende Di- und/oder Polyisocyanate. Im einzelnen kommen beispielsweise in Betracht Urethangruppen enthaltende organische, vorzugsweise aromatische Polyisocyanate mit NCO-Gehalten von 33 bis 15 Gew.-%, vorzugsweise von 31 bis 21 Gew.-%, bezogen auf das Gesamtgewicht des Polyisocyanats.

Die nach dem erfindungsgemäßen Verfahren hergestellten Polyole können in Kombination mit anderen Verbindungen mit mindestens zwei mit Isocyanatgruppen reaktiven Wasserstoffatomen eingesetzt werden.

Als Verbindungen mit mindestens zwei mit Isocyanat reaktiven Wasserstoffatomen, die gemeinsam mit den nach dem erfindungsgemäßen Verfahren hergestellten Polyolen verwendet werden können, kommen insbesondere Polyetheralkohole und/oder Polyesteralkohole zum Einsatz.

Bei der Herstellung von Polyurethan-Hartschaumstoffen wird zumeist mindestens ein Polyetheralkohol eingesetzt, der eine Funktionalität von mindestens 4 und eine Hydroxylzahl größer 250 mgKOH/g aufweist.

Die gemeinsam mit den nach dem erfindungsgemäßen Verfahren hergestellten Polyolen eingesetzten Polyesteralkohole werden zumeist durch Kondensation von mehrfunktionellen Alkoho-len, vorzugsweise Diolen, mit 2 bis 12 Kohlenstoffatomen, vorzugsweise 2 bis 6 Kohlenstoffatomen, mit mehrfunktionellen Carbonsäuren mit 2 bis 12 Kohlenstoffatomen, beispielsweise Bernsteinsäure, Glutarsäure, Adipinsäure, Korksäure, Azelainsäure, Sebacinsäure, Decandicarbonsäure, Maleinsäure, Fumarsäure und vorzugsweise Phthalsäure, Isophthalsäure, Terephthalsäure und die isomeren Naphthalindicarbonsäuren, hergestellt.

Die gemeinsam mit den nach dem erfindungsgemäßen Verfahren hergestellten Polyolen verwendeten Polyetheralkohole haben zumeist eine Funktionalität zwischen 2 und 8, insbesondere 4 bis 8.

Insbesondere als Polyhydroxylverbindungen verwendet werden Polyetherpolyole, die nach bekannten Verfahren, beispielsweise durch anionische Polymerisation von Alkylenoxiden in Gegenwart von Alkalimetallhydroxiden, hergestellt werden.

Als Alkylenoxide werden vorzugsweise Ethylenoxid und 1,2-Propylenoxid eingesetzt. Die Alkylenoxide können einzeln, alternierend nacheinander oder als Mischungen verwendet werden.

Als Startermoleküle kommen beispielsweise in Betracht: Wasser, organische Dicarbonsäuren, wie z.B. Bernsteinsäure, Adipinsäure, Phthalsäure und Terephthalsäure, aliphatische und aromatische, gegebenenfalls N-mono-, N,N- und N,N'-dialkyl-substituierte Diamine mit 1 bis 4 Kohlenstoffatomen im Alkylrest, wie z.B. gegebenenfalls mono- und dialkylsubstituiertes Ethylendiamin, Diethylentriamin, Triethylentetramin, 1,3-Propylendiamin, 1,3- bzw. 1,4-Butylendiamin, 1,2-, 1,3-, 1,4-, 1,5- und 1,6-Hexamethylendiamin, Anilin, Phenylendiamine, 2,3-, 2,4-, 3,4- und 2,6-Toluylendiamin und 4,4'-, 2,4'- und 2,2'-Diaminodiphenylmethan.

Als Startermoleküle kommen ferner in Betracht: Alkanolamine, wie z.B. Ethanolamin, N-Methyl- und N-Ethylethanolamin, Dialkanolamine, wie z.B. Diethanolamin, N-Methyl- und N-Ethyldiethanolamin und Trialkanolamine wie z.B. Triethanolamin und Ammoniak.

Weiterhin eingesetzt werden mehrwertige, insbesondere zwei- und/oder dreiwertige Alkohole, wie Ethandiol, Propandiol-1,2 und -1,3, Diethylenglykol, Dipropylenglykol, Butandiol-1,4, Hexandiol-1,6, Glycerin, Pentaerythrit, Sorbit und Saccharose, mehrwertige Phenole, wie z.B. 4,4'-Dihydroxydiphenylmethan und 4,4'-Dihydroxydiphenylpropan-2,2, Resole, wie z.B. oligomere Kondensationsprodukte aus Phenol und Formaldehyd und Mannich-Kondensate aus Phenolen, Formaldehyd und Dialkanolaminen sowie Melamin.

Die Polyetherpolyole besitzen eine Funktionalität von vorzugsweise 3 bis 8 und insbesondere 3 und 6 und Hydroxylzahlen von vorzugsweise 120 mgKOH/g bis 770 mgKOH/g und insbesondere 240 mgKOH/g bis 570 mgKOH/g.

Zu den Verbindungen mit mindestens zwei mit Isocyanatgruppen reaktiven Wasserstoffatomen gehören auch die gegebenenfalls mitverwendeten Kettenverlängerer und Vernetzer. Zur Modifizierung der mechanischen Eigenschaften kann sich jedoch der Zusatz von difunktionellen Kettenverlängerungsmitteln, tri- und höherfunktionellen Vernetzungsmitteln oder gegebenenfalls auch Gemischen davon als vorteilhaft erweisen. Als Kettenverlängerungs- und/oder Vernetzungsmittel verwendet werden vorzugsweise Alkanolamine und insbesondere Diole und/oder Triole mit Molekulargewichten kleiner als 400, vorzugsweise 60 bis 300.

Sofern zur Herstellung der Polyurethane Kettenverlängerungsmittel, Vernetzungsmittel oder Mischungen davon Anwendung finden, kommen diese zweckmäßigerweise in einer Menge von 0 bis 20 Gew.-%, vorzugsweise 2 bis 5 Gew.-%, bezogen auf das Gewicht der Verbindungen mit mindestens zwei mit Isocyanatgruppen reaktiven Wasserstoffatomen, zum Einsatz.

Als Treibmittel kann beispielsweise Wasser verwendet werden, das mit Isocyanatgruppen unter Abspaltung von Kohlendioxid reagiert. An Stelle von, vorzugsweise jedoch in Kombination mit Wasser können auch sogenannte physikalische Treibmittel eingesetzt werden. Dabei handelt es sich um gegenüber den Einsatzkomponenten inerte Verbindungen, die zumeist bei Raumtemperatur flüssig sind und unter den Bedingungen der Urethanreaktion verdampfen. Vorzugsweise liegt der Siedepunkt dieser Verbindungen unter 110°C, insbesondere unter 80 °C. Zu den physikalischen Treibmitteln zählen auch inerte Gase, die in den in die Einsatzkomponenten eingebracht bzw. in ihnen gelöst werden, beispielsweise Kohlendioxid, Stickstoff oder Edelgase.

Die bei Raumtemperatur flüssigen Verbindungen werden zumeist ausgewählt aus der Gruppe, enthaltend Alkane und/oder Cycloalkane mit mindestens 4 Kohlenstoffatomen, Dialkylether, Ester, Ketone, Acetale, Fluoralkane mit 1 bis 8 Kohlenstoffatomen, und Tetraalkylsilane mit 1 bis 3 Kohlenstoffatomen in der Alkylkette, insbesondere Tetramethylsilan.

Als Beispiele seien genannt Propan, n-Butan, iso- und Cyclobutan , n-, iso- und Cyclopentan, Cyclohexan, Dimethylether, Methylethylether, Methylbutylether, Ameisensäuremethylester, Aceton, sowie Fluoralkane, die in der Troposphäre abgebaut werden können und deshalb für die Ozonschicht unschädlich sind, wie Trifluormethan, Difluormethan, 1,1,1,3,3-Pentafluorbutan, 1,1,1,3,3-Pentafluorpropan, 1,1,1,2-Tetrafluorethan, Difluorethan und Heptafluorpropan. Die genannten physikalischen Treibmittel können allein oder in beliebigen Kombinationen untereinander eingesetzt werden.

Als Katalysatoren werden insbesondere Verbindungen eingesetzt, welche die Reaktion der Isocyanatgruppen mit den mit Isocyanatgruppen reaktiven Gruppen stark beschleunigen. Insbesondere eingesetzt werden organische Metallverbindungen, vorzugsweise organische Zinnverbindungen, wie Zinn(II)-salze von organischen Säuren.

Weiterhin können als Katalysatoren stark basische Amine eingesetzt werden. Beispiele hierfür sind sekundäre aliphatische Amine, Imidazole, Amidine, Triazine sowie Alkanolamine.

Die Katalysatoren können, je nach Erfordernis, allein oder in beliebigen Mischungen untereinander eingesetzt werden.

Als Hilfsmittel und/oder Zusatzstoffe kommen die für diesen Zweck an sich bekannten Stoffe, beispielsweise oberflächenaktive Substanzen, Schaumstabilisatoren, Zellregler, Füllstoffe, Pigmente, Farbstoffe, Flammschutzmittel, Hydrolyseschutzmittel, Antistatika, fungistatisch und bakteriostatisch wirkende Mittel zum Einsatz.

Nähere Angaben über die zur Durchführung des erfindungsgemäßen Verfahrens eingesetzten Ausgangsstoffe, Treibmittel, Katalysatoren sowie Hilfs- und/oder Zusatzstoffe finden sich beispielsweise im Kunststoffhandbuch, Band 7, "Polyurethane" Carl-Hanser-Verlag München, 1. Auflage, 1966, 2. Auflage, 1983 und 3. Auflage, 1993.

Der Vorteil des erfindungsgemäßen Verfahrens gegenüber der Epoxidierung/Ringöffnung bzw. der Hydroformylierung/Hydrierung besteht darin, dass für den Ketonisierungs-Prozess keine Lösungsmittel und keine Katalysatoren benötigt werden. Damit ist ein vergleichsweise kostengünstiger Zugang hydroxyfunktionalisierter Fette bzw. Fettsäurederivate möglich. Zusätzlich besteht der Vorteil, dass durch einfache Anpassung der Reaktionsbedingungen wie Druck, Temperatur und Verweilzeit sehr einfach und gezielt Funktionalitäten eingestellt werden können und somit Materialien zugänglich werden, die sehr breite Anwendungsmöglichkeiten bieten, die auch über Polyurethananwendungen hinaus gehen.

Gegenüber der Epoxidierung und der Ozonolyse bietet dieses Verfahren den Vorteil, Oligohydroxyfette zu generieren, die bei frei einstellbaren Hydroxylierunggrad keine Doppelbindungen mehr enthalten und somit dem gewöhnlichen Alterungsprozess von Fetten nicht mehr unterliegen (Oxidation der DB, "Ranzigwerden"). Im Falle der Epoxidierung bzw. Ozonolyse gelingt dies nur bei vollständigem Umsatz, dies legt jedoch den Funktionalisierungsgrad fest.

Im Vergleich zur Hydroformylierung ermöglicht die Lachgasoxidation die Herstellung von Material mit komplementärer Reaktivität, da hier ausschließlich sekundäre Hydroxygruppen erzeugt werden, während die Hydroformylierung primäre OH-Gruppen erzeugt.

Durch die nachfolgende Anlagerung der Alkylenoxide können die Polyole für ihren jeweiligen Einsatzzweck optimiert werden. so können bei Polyolen, die für den Einsatz in Polyurethan-Weichschaumstoffen vorgesehen sind, längere Ketten angelagert werden, als bei solchen für den Einsatz in Polyurethan-Hartschaumstoffen.

Die Erfindung soll an den nachfolgenden Beispielen näher erläutert werden.

### Beispiel 1: Oxidation von Sojaöl mit Lachgas

In einen Stahlautoklaven mit 1,2 L Fassungsvolumen wurden 260 g Sojaöl gefüllt, der Autoklav verschlossen und mit Stickstoff inertisiert. 50 bar Lachgas wurden aufgepresst, der Rührer auf 700 U/min eingestellt und eingeschaltet und in Folge die Reaktionsmischung auf 220 °C erwärmt. Nach 22 h Laufzeit wurde auf Raumtemperatur abgekühlt, der Rührer ausgeschaltet und langsam auf Umgebungsdruck entspannt. Nach der Entfernung des Lösungsmittels wurde der gelbliche flüssige Austrag analysiert. Analytische Daten: Bromzahl 36 g Brom/100g, Carbonylzahl 173 mg KOH/g, Esterzahl 196 mg KOH/g, Säurezahl 1,8 mg KOH/g. Elementaranalyse: C = 73,6%, H = 10,8%, O = 15,1%.

### Beispiel 2: Oxidation von Sojaöl mit Lachgas

In einen Stahlautoklaven mit 1,2 L Fassungsvolumen wurden 172 g Sojaöl und 172 g Cyclohexan gefüllt, der Autoklav verschlossen und mit Stickstoff inertisiert. 20 bar Lachgas wurden aufgepresst, der Rührer auf 700 U/min eingestellt und eingeschaltet und in Folge die Reaktionsmischung auf 220 °C erwärmt. Nach 36 h Laufzeit wurde auf Raumtemperatur abgekühlt, der Rührer ausgeschaltet und langsam auf Umgebungsdruck entspannt. Nach der Entfernung des Lösungsmittels wurde der gelbliche flüssige Austrag analysiert.

Analytische Daten: Bromzahl 57 g Brom/100g, Carbonylzahl 64 mg KOH/g, Esterzahl 196 mg KOH/g, Säurezahl 1,8 mg KOH/g. Elementaranalyse: C = 75,6%, H = 11,5%, O = 13,4%.

### Beispiel 3: Oxidation von Sojaöl mit Lachgas im Rohrreaktor

In einem Rohrreaktor (Innenvolumen 210 mL, Verweilzeit ca. 50 min) wurden bei 290 °C und 100 bar 130 g/h einer Mischung aus 50 Gew% Sojaöl und 50 Gew% Cyclohexan mit 45 g/h Lachgas zur Reaktion gebracht. Der Reaktionsaustrag wurde in einen Behälter entspannt, der flüssige Anteil des Reationaustrages abgekühlt und das Cyclohexan destillativ entfernt. Der gelbliche flüssige Austrag wurde analysiert. Analytische Daten: Bromzahl 54 g Brom/100g, Carbonylzahl 81 mg KOH/g, Esterzahl 199 mg KOH/g, Säurezahl 2,6 mg KOH/g. Elementaranalyse: C = 75,0%, H = 11,1 %, O = 13,7%.

Als Sojaöl wurde in allen Beispielen ein kommerzielles Produkt der Fa. Aldrich mit einer Bromzahl von 80 g Brom/100g, einer Carbonylzahl von 1 mg KOH/100G, einer Verseifungszahl von 192 mg KOH/g und einer Säurezahl von <0,1 mg KOH/g eingesetzt. Die Elementaranalyse ergab C = 77,6%, H = 11,7%, O = 11,0%.

### Beispiel 4: Hydrierung des oxidierten Sojaöls aus Beispiel 2

In einem 300 mL Stahlautoklaven wird eine Lösung von 20 g oxidiertem Sojaöl aus Beispiel 2 (Carbonylzahl = 64, OH-Zahl < 5, Bromzahl = 57) in 100 mL Tetrahydrofuran zusammen mit 2 g eines wasserfeuchten, 5%igen Ruthenium-Katalysators auf einem Kohlenstoffträger vorgelegt. Es wurde auf 120°C erhitzt und 120 bar Wasserstoff wurden aufgepresst. Bei diesen Parametern wurde 12 h lang gerührt. Anschließend wurde die Reaktionsmischung abgekühlt und entspannt. Der Austrag wurde filtriert und das Lösemittel wird destillativ entfernt. Die Analyse des festen (butterartigen) Rückstands ergab eine OH-Zahl von 64, eine Carbonylzahl <5 und eine Bromzahl von <5.

### Beispiel 5: Hydrierung des oxidierten Sojaöls aus Beispiel 3

In einem 300 mL Stahlautoklaven wurde eine Lösung von 20 g oxidiertem Sojaöl (Carbonylzahl = 81, Bromzahl = 54) in 100 mL Tetrahydrofuran zusammen mit 20 g eines wasserfeuchten, auf Al₂O₃ geträgerten Ruthenium-Katalysator (0,5%ig) vorgelegt. Es wurde auf 120°C erhitzt und 100 bar Wasserstoff wurden aufgepresst. Bei diesen Parametern wurde 12 h lang gerührt. Anschließend wurde die Reaktionsmischung abgekühlt und entspannt. Der Reaktionsaustrag wurde filtriert und danach das Lösemittel destillativ entfernt. Die Analyse des festen (butterartigen) Rückstands ergab eine OH-Zahl von 80, eine Carbonylzahl <5 und eine Bromzahl von <5.

### Beispiel 6: Hydrierung des oxidierten Sojaöls aus Beispiel 1

In einem 300 mL Stahlautoklaven wurde eine Lösung von 20 g oxidiertem Sojaöl aus Beispiel 1 (Carbonylzahl = 173, OH-Zahl < 5, Bromzahl = 36) in 100 mL Tetrahydrofuran zusammen mit 2 g eines wasserfeuchten, 5%igen Ruthenium-Katalysators auf einem Kohlenstoffträger vorgelegt. Es wurde auf 120°C erhitzt und 120 bar Wasserstoff wurden aufgepresst. Bei diesen Parametern wurde 12 h lang gerührt. Anschließend wurde die Reaktionsmischung abgekühlt und entspannt. Der Austrag wurde filtriert und danach das Lösemittel destillativ entfernt. Die Analyse des festen (butterartigen) Rückstands ergab eine OH-Zahl von 170, eine Carbonylzahl <5 und eine Bromzahl von <5.

Das Polyol aus Beispiel 6 wurde in einer Polyurethan-Hartschaum-Rezeptur eingesetzt. Dabei wurde festgestellt, dass sich das System durch eine ausgezeichnete Verträglichkeit mit dem als Treibmittel eingesetzten Pentan auszeichnete.

### Beispiel 7: Alkoxylierung von Hydroxy-Sojaöl aus Beispiel 6

1523 g Hydroxyöl aus Beispiel 6 (OH-Zahl = 170 mg KOH/g) wurden in einem Druckautoklaven vorgelegt und mit 11,5 g einer 5,4%igen Suspension eines Zinkhexacyanocobaltats in Lupranol® 1100 versetzt. Nachdem die Reaktionsmischung dreimal mit Stickstoff inertisiert wurde, wurde die Reaktionsmischung unter Vakuum bei 20 mbar für ca. 30 Minuten bei 130°C vom Wasser befreit. Anschließend wurden zunächst zur Aktivierung des Katalysators 150 g Propylenoxid zur Reaktionsmischung innerhalb von 10 Minuten dosiert. Nach der Aktivierung, die sich durch eine Temperaturerhöhung in Kombination mit einem signifikanten Druckabfall bemerkbar machte, wurden weitere 3720 g Propylenoxid zur Reaktionsmischung innerhalb von 160 Minuten dosiert. Nach beendeter Monomerdosierung und nach Erreichen eines konstanten Reaktordruckes wurden nicht umgesetztes Propylenoxid und andere flüchtige Bestandteile im Vakuum abdestilliert und das Produkt abgelassen. Es wurden so 5300 g des gewünschten Produktes in Form einer schwach gelblichen, viskosen Flüssigkeit mit einer OH-Zahl von 50,6 mg KOH/g und einer Viskosität 842 mPas erhalten.

Das Polyol aus Beispiel 7 wurde in einer Polyurethan-Weichschaum-Rezeptur eingesetzt. Dabei wurde das Polyol als einziges Polyol eingesetzt. Es gab keinerlei negative Auswirkungen auf die Prozessierbarkeit des System oder auf die mechanischen Parameter des Weichschaums.

### Beispiel 8: Alkoxylierung von Hydroxy-Sojaöl aus Beispiel 5

917 g Hydroxyöl aus Beispiel 5 (OH-Zahl = 80 mg KOH/g) wurden in einem Druckautoklaven vorgelegt und mit 6,42 g einer 5,7%igen Suspension eines Zinkhexacyanocobaltats in Lupranol® 1100 versetzt. Nachdem die Reaktionsmischung dreimal mit Stickstoff inertisiert wurde, wurde die Reaktionsmischung unter Vakuum bei 20 mbar für ca. 30 Minuten bei 130°C vom Wasser befreit. Anschließend wurden zunächst zur Aktivierung des Katalysators 50 g Propylenoxid zur Reaktionsmischung innerhalb von 10 Minuten dosiert. Nach der Aktivierung, die sich durch eine Temperaturerhöhung in Kombination mit einem signifikanten Druckabfall bemerkbar machte, wurden weitere 500 g Propylenoxid zur Reaktionsmischung innerhalb von 100 Minuten dosiert. Nach beendeter Monomerdosierung und nach Erreichen eines konstanten Reaktordruckes wurden nicht umgesetztes Propylenoxid und andere flüchtige Bestandteile im Vakuum abdestilliert und das Produkt abgelassen. Es wurden so 1350 g des gewünschten Produktes in Form einer schwach gelblichen, viskosen Flüssigkeit mit einer OH-Zahl von 49,8 mg KOH/g und einer Viskosität 527 mPas erhalten.

Das Polyol aus Beispiel 8 wurde in einer Polyurethan-Mittelschuhsohlen-Rezeptur eingesetzt Dabei wurde das Polyol als einziges Polyol eingesetzt. Die erhaltenen Produkte zeichnen sich zudem durch eine verbesserte Oberflächenbeschaffenheit aus. Das Polyol aus Beispiel 8 wurde weiterhin in einer Polyurethan-Dichtstoff-Rezeptur eingesetzt. Die erhaltenen Dichtstoffe zeichneten sich durch ausgezeichnete Hydrolysestabilitäten aus.

## Patentansprüche

1. Verfahren zur Herstellung von Polyolen, umfassend die Schritte
a) Umsetzung von ungesättigten natürlichen Fetten, ungesättigten natürlichen Fettsäuren und/oder Fettsäureestern mit Distickstoffmonoxid,
b) Umsetzung des in Schritt a) erhaltenen Produkts mit einem Hydrierungsreagenz
c) Umsetzung des Reaktionsprodukts aus Schritt b) mit Alkylenoxiden.

2. Verfahren nach Anspruch 1, **dadurch gekennzeichnet, dass** die ungesättigten natürlichen Fette sowie Fettderivate ausgewählt sind aus der Gruppe, enthaltend Rizinusöl, Traubenkernöl, Schwarzkümmelöl, Kürbiskernöl, Borretschsamenöl, Sojaöl, Weizenkeimöl, Rapsöl, Sonnenblumenöl, Erdnussöl, Aprikosenkernöl, Pistazienkemöl, Mandelöl, Olivenöl, Macadamianussöl, Avocadoöl, Sanddomöl, Sesamöl, Hanföl, Haselnussöl, Nachtkerzenöl, Wildrosenöl, Distelöl, Walnussöl, Palmöl, Fischöl, Kokosnussöl, Tallöl, Maiskeimöl, Leinsamenöl.

3. Verfahren nach Anspruch 1, **dadurch gekennzeichnet, dass** die Fettsäuren und Fettsäureester ausgewählt sind aus der Gruppe, enthaltend Myristoleinsäure, Palmitoleinsäure, Olsäure, Vaccensäure, Petroselinsäure, Gadoleinsäure, Erucasäure, Nervonsäure, Linolsäure, α- und γ-Linolensäure, Stearidonsäure, Arachidonsäure, Timnodonsäure, Clupanodonsäure und Cervonsäure, sowie deren Estern.

4. Verfahren nach Anspruch 1, **dadurch gekennzeichnet, dass** die ungesättigten natürlichen Fette ausgewählt sind aus der Gruppe, enthaltend Sojaöl, Palmöl, Sonnenblumenöl und Rapsöl.

5. Verfahren nach Anspruch 1, **dadurch gekennzeichnet, dass** in Schritt a) das Lachgas im Gemisch mit inerten Gasen eingesetzt wird.

6. Verfahren nach Anspruch 1, **dadurch gekennzeichnet, dass** das Hydrierungsreagenz ein komplexes Metallhydrid ist.

7. Verfahren nach Anspruch 1, **dadurch gekennzeichnet, dass** das Hydrierungsreagenz Lithiumaluminiumhydrid oder Natriumborhydrid oder Lithiumborhydrid ist.

8. Verfahren nach Anspruch 1, **dadurch gekennzeichnet, dass** das Hydrierungsreagenz Wasserstoff ist.

9. Verfahren nach Anspruch 8, **dadurch gekennzeichnet, dass** Schritt b) in Anwesenheit eines Katalysators durchgeführt wird.

10. Verfahren nach Anspruch 8, **dadurch gekennzeichnet, dass** Schritt b) in Anwesenheit eines Katalysators durchgeführt wird, der zumindest ein Übergangsmetall der Gruppen 6 bis 11 enthält.

11. Verfahren nach Anspruch 8, **dadurch gekennzeichnet, dass** Schritt b) in Anwesenheit eines Ruthenium enthaltenen Katalysators durchgeführt wird.

12. Verfahren nach Anspruch 8, **dadurch gekennzeichnet, dass** Schritt b) in Anwesenheit eines Nickel enthaltenen Katalysators durchgeführt wird.

13. Verfahren nach Anspruch 1, **dadurch gekennzeichnet, dass** die Anlagerung der Alkylenoxide in Schritt c) in Anwesenheit eines Katalysators durchgeführt wird.

14. Verfahren nach Anspruch 1, **dadurch gekennzeichnet, dass** die Anlagerung der Alkylenoxide in Schritt c) in Anwesenheit eines Multimetallcyanid-Katalysators durchgeführt wird.

15. Verfahren zur Herstellung von Polyurethanen durch Umsetzung von Polyisocyanaten mit Verbindungen mit zwei mit Isocyanatgruppen reaktiven Wasserstoffatomen, **dadurch gekennzeichnet, dass** als Verbindungen mit mindestens zwei mit Isocyanatgruppen reaktiven Wasserstoffatomen nach einem der Ansprüche 1 bis 14 hergestellte Polyole eingesetzt werden.

## Claims

1. A method for producing polyols, comprising the steps
a) reacting unsaturated natural fats, unsaturated natural fatty acids and/or fatty acid esters with dinitrogen monoxide,
b) reacting the product obtained in step a) with a hydrogenation reagent
c) reacting the reaction product from step b) with alkylene oxides.

2. The method according to claim 1, wherein the unsaturated natural fats and also fatty derivatives are selected from the group comprising castor oil, grapeseed oil, black caraway oil, pumpkin seed oil, borage seed oil, soya oil, wheat germ oil, rapeseed oil, sunflower oil, peanut oil, apricot kernel oil, pistachio kernel oil, almond oil, olive oil, macadamia nut oil, avocado oil, sea buckthorn oil, sesame oil, hemp oil, hazelnut oil, evening primrose oil, wild rose oil, safflower oil, walnut oil, palm oil, fish oil, coconut oil, tall oil, corn germ oil, linseed oil.

3. The method according to claim 1, wherein the fatty acids and fatty acid esters are selected from the group comprising myristoleic acid, palmitoleic acid, oleic acid, vaccenic acid, petroselinic acid, gadoleic acid, erucic acid, nervonic acid, linoleic acid, α- and γ-linolenic acid, stearidonic acid, arachidonic acid, timnodonic acid, clupanodonic acid and cervonic acid, and esters thereof.

4. The method according to claim 1, wherein the unsaturated natural fats are selected from the group comprising soya oil, palm oil, sunflower oil and rapeseed oil.

5. The method according to claim 1, wherein, in step a), the nitrous oxide is used in a mixture with inert gases.

6. The method according to claim 1, wherein the hydrogenation reagent is a complex metal hydride.

7. The method according to claim 1, wherein the hydrogenation reagent is lithium aluminum hydride or sodium borohydride or lithium borohydride.

8. The method according to claim 1, wherein the hydrogenation reagent is hydrogen.

9. The method according to claim 8, wherein step b) is carried out in the presence of a catalyst.

10. The method according to claim 8, wherein step b) is carried out in the presence of a catalyst which comprises at least one transition metal of groups 6 to 11.

11. The method according to claim 8, wherein step b) is carried out in the presence of a ruthenium-comprising catalyst.

12. The method according to claim 8, wherein step b) is carried out in the presence of a nickel-comprising catalyst.

13. The method according to claim 1, wherein the addition reaction of the alkylene oxides in step c) is carried out in the presence of a catalyst.

14. The method according to claim 1, wherein the addition reaction of the alkylene oxides in step c) is carried out in the presence of a multi-metal cyanide catalyst.

15. A method for producing polyurethanes by reacting polyisocyanates with compounds having two hydrogen atoms that are reactive with isocyanate groups, wherein polyols produced according to any of claims 1 to 14 are used as compounds having at least two hydrogen atoms that are reactive with isocyanate groups.

## Revendications

1. Procédé pour la préparation de polyols, comprenant les étapes
a) mise en réaction de graisses naturelles insaturées, d'acides gras naturels insaturés et/ou d'esters d'acides gras avec du protoxyde d'azote,
b) mise en réaction du produit obtenu dans l'étape a) avec un réactif d'hydrogénation
c) mise en réaction du produit de réaction provenant de l'étape b) avec des oxydes d'alkylène.

2. Procédé selon la revendication 1, **caractérisé en ce que** les graisses naturelles insaturées ainsi que les dérivés de graisses sont choisis dans le groupe contenant l'huile de ricin, l'huile de pépins de raisin, l'huile de nigelle, l'huile de graines de courge, l'huile de graines de bourrache, l'huile de soja, l'huile de germes de blé, l'huile de colza, l'huile de tournesol, l'huile d'arachide, l'huile de noyaux d'abricot, l'huile de pistache, l'huile d'amande, l'huile d'olive, l'huile de noix de macadamia, l'huile d'avocat, l'huile de graines d'argousier, l'huile de sésame, l'huile de chènevis, l'huile de noisette, l'huile d'onagre, l'huile d'églantine, l'huile de carthame, l'huile de noix, l'huile de palme, l'huile de poisson, l'huile de coprah, le tallöl, l'huile de germes de maïs, l'huile de lin.

3. Procédé selon la revendication 1, **caractérisé en ce que** les acides gras et esters d'acides gras sont choisis dans le groupe contenant l'acide myristoléique, l'acide palmitoléique, l'acide oléique, l'acide vaccénique, l'acide pétrosélinique, l'acide gadoléique, l'acide érucique, l'acide nervonique, l'acide linoléique, l'acide α- et γ-linolénique, l'acide stéaridonique, l'acide arachidonique, l'acide timnodonique, l'acide clupanodonique et l'acide cervonique, ainsi que leurs esters.

4. Procédé selon la revendication 1, **caractérisé en ce que** les graisses naturelles insaturées sont choisies dans le groupe contenant l'huile de soja, l'huile de palme, l'huile de tournesol et l'huile de colza.

5. Procédé selon la revendication 1, **caractérisé en ce que** dans l'étape a) on utilise le gaz hilarant en mélange avec des gaz inertes.

6. Procédé selon la revendication 1, **caractérisé en ce que** le réactif d'hydrogénation est un hydrure métallique complexe.

7. Procédé selon la revendication 1, **caractérisé en ce que** le réactif d'hydrogénation est l'hydrure de lithium et d'aluminium ou le borohydrure de sodium ou le borohydrure de lithium.

8. Procédé selon la revendication 1, **caractérisé en ce que** le réactif d'hydrogénation est l'hydrogène.

9. Procédé selon la revendication 8, **caractérisé en ce que** l'étape b) est effectuée en présence d'un catalyseur.

10. Procédé selon la revendication 8, **caractérisé en ce que** l'étape b) est effectuée en présence d'un catalyseur qui contient au moins un métal de transition des groupes 6 à 11.

11. Procédé selon la revendication 8, **caractérisé en ce que** l'étape b) est effectuée en présence d'un catalyseur contenant du ruthénium.

12. Procédé selon la revendication 8, **caractérisé en ce que** l'étape b) est effectuée en présence d'un catalyseur contenant du nickel.

13. Procédé selon la revendication 1, **caractérisé en ce que** la fixation par addition des oxydes d'alkylène dans l'étape c) est effectuée en présence d'un catalyseur.

14. Procédé selon la revendication 1, **caractérisé en ce que** la fixation par addition des oxydes d'alkylène dans l'étape c) est effectuée en présence d'un catalyseur de type cyanure multimétallique.

15. Procédé pour la production de polyuréthanes par mise en réaction de polyisocyanates avec des composés comportant deux atomes d'hydrogène réactifs avec des groupes isocyanate, **caractérisé en ce que** comme composés comportant au moins deux atomes d'hydrogène réactifs avec des groupes isocyanate on utilise des polyols préparés selon l'une quelconque des revendications 1 à 14.
